(19) Europäisches Patentamt
European Patent Office
Office européen des brevets

(11) Numéro de publication: **0 297 958**
**A1**

(12) # DEMANDE DE BREVET EUROPEEN

(21) Numéro de dépôt: **88401608.0**

(51) Int. Cl.[4]: **A 61 K 35/14**

(22) Date de dépôt: **24.06.88**

(30) Priorité: **25.06.87 FR 8708964**

(43) Date de publication de la demande:
**04.01.89 Bulletin 89/01**

(84) Etats contractants désignés:
**AT BE CH DE ES FR GB GR IT LI LU NL SE**

(71) Demandeur: **FONDATION NATIONALE DE TRANSFUSION SANGUINE**
**6, rue Alexandre Cabanel**
**F-75739 Paris Cédex 15 (FR)**

**CENTRE HOSPITALIER REGIONAL DE TOURS**
**2, Boulevard Tonnelé**
**F-37044 Tours Cédex (FR)**

(72) Inventeur: **Pernelle, Michel René**
**La Ganneraye**
**F-37300 Joue les Tours (FR)**

**Ropars, Claude**
**16, rue de la Pinterie**
**F-37170 Saint Avertin (FR)**

(74) Mandataire: **Warcoin, Jacques et al**
**Cabinet Régimbeau 26, avenue Kléber**
**F-75116 Paris (FR)**

(54) **Procédé de stabilisation de membranes cellulaires et son application à la conservation de cellules sous une forme protégeant l'essentiel de leurs antigènes de surface.**

(57) La présente invention concerne un procédé de stabilisation des membranes cellulaires, en vue notamment de conserver les cellules sous une forme protégeant l'essentiel de leurs antigènes de surface, caractérisé en ce que lesdites membranes sont traitées par un agent stabilisant au moins bifonctionnel susceptible de les rigidifier, ledit agent stabilisant pouvant être disloqué ou éliminé dans des conditions permettant de récupérer les membranes cellulaires ayant conservé l'essentiel de leurs propriétés biologiques ou antigéniques.

Application à la congélation, à la lyophilisation et/ou à la conservation des cellules, stromas cellulaires ou membranes.

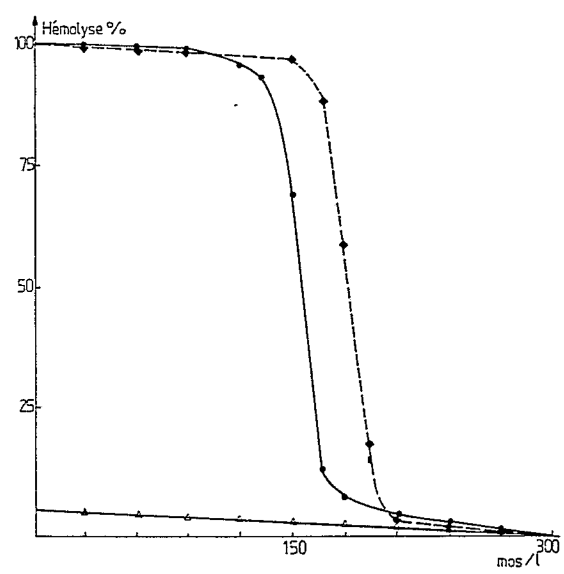


EP 0 297 958 A1

**Description**

**PROCEDE DE STABILISATION DE MEMBRANES CELLULAIRES ET SON APPLICATION A LA CONSERVATION DE CELLULES SOUS UNE FORME PROTEGEANT L'ESSENTIEL DE LEURS ANTIGENES DE SURFACE**

La présente invention concerne un procédé de stabilisation réversible de la membrane de diverses cellules, et, en particulier, des cellules sanguines en vue notamment de les conserver sous une forme protégeant l'essentiel de leurs propriétés biologiques et antigéniques.

La conservation des cellules sanguines est un problème très important en hématologie et pour la transfusion sanguine. Un grand nombre de solutions et de perfectionnements ont été apportés à ce problème, mais ils sont encore insuffisants et la conservation de ces éléments cellulaires est soumises à de nombreuses limitations.

Il faut considérer essentiellement trois solutions différentes : la conservation en milieu liquide, la cryoprotection, la lyophilisation.

La conservation en milieu liquide, la plus connue, fait appel à des solutions anticoagulantes, des solutions de conservation et des solutions de régénération. Les globules rouges peuvent être ainsi conservés entre 30 et 50 jours pour un usage transfusionnel. Par contre, la stabilité des globules tests constituant les panels de dépistage est actuellement limitée à 3-4 semaines, une conservation prolongée s'accompagnant d'une diminution des activités antigéniques de la membrane. Au-delà, une hémolyse importante apparaît progressivement.

La conservation par congélation, est la méthode de choix pour la conservation prolongée des globules rouges et autres cellules. Elle fait appel à des cryoprotecteurs dits internes, car traversant la membrane cellulaire, comme le glycérol ou le diméthylsulfoxyde, ou externes, généralement des macromolécules (PVP, dextrane, etc.). Les températures varient entre celles de l'azote liquide et -25°C.

Il est possible de congeler de la même façon des cellules comme la plupart des globules blancs dans des conditions satisfaisantes. Par contre, la congélation de certaines cellules du type granulocytes n'est pas actuellement possible, car elles sont soumises à des chocs osmotiques importants dans les protocoles de congélation-décongélation.

Les techniques de congélation nécessitent des installations fixes importantes qui limitent les possibilités d'utilisation de produits congelés, en particulier pour les éléments cellulaires réactifs dont le transport est possible mais difficile.

La conservation par lyophilisation - il s'agit d'une méthode idéale théoriquement pour la conservation de cellules, mais qui, actuellement, n'est pas utilisable malgré diverses tentatives (H.T. Meryman. Drying of Living Mammalian Cells, Anti N.Y. Acad. Sci. (1960), 729-734). Essentiellement, les processus de congélation, déshydratation et réhydratation s'accompagnent d'une série de phénomènes de dégradation liés, d'une part, à la formation de cristaux de glace dans la cellule, et, d'autre part, à des chocs osmotiques successifs subis en particulier par la membrane cellulaire lorsque des lyoprotecteurs internes ou externes sont utilisés. Les érythrocytes peuvent être stabilisés durablement avant lyophilisation, à l'aide de divers réactifs (formol, acide tannique, dialdéhyde, chrome $Cr^{3+}$, etc.). Ces molécules rigidifient la membrane de sorte que la cellule n'est plus sensible aux chocs osmotiques. Cependant, la rigidification membranaire conduit à des particules inertes ayant perdu la plupart de leurs caractères antigéniques propres ainsi que leur mobilité membranaire. Ces particules sont utilisées, une fois recouvertes de molécules d'antigènes exogènes, dans des réactions dites d'hémagglutination passive ou d'inhibition de l'hémagglutination passive (D.H. Bing et al., Exp. Biol. Med. (1967) 124, 1166, 70 et H. Itto et al., J. Clin. Microbiol. (1976), 4, 188-189).

En outre, la réaction d'hémagglutination érythrocytaire met en jeu, d'une part la fixation spécifique d'un anticorps sur un antigène de la membrane, suivie d'une modification des propriétés physicochimiques de la membrane (charge ionique, constante diélectrique), et, d'autre part, la formation d'un réseau antigène-anticorps directement lié à la mobilité et la déformabilité membranaire).

La perte de la mobilité membranaire liée à une réticulation s'accompagne donc de la disparition des réactions d'hémagglutination liées aux antigènes de groupe sanguin. De ce fait, il n'existe pas actuellement de panels érythrocytaires sous forme stabilisée et/ou lyophilisée. Il en est de même pour les panels de lymphocytes destinés à la détection des antigènes d'histocompatibilité.

La présente invention concerne un nouveau procédé de stabilisation des membranes cellulaires, en vue notamment de conserver les cellules, leurs membranes ou leurs stromas sous une forme protégeant l'essentiel de leurs antigènes de surface, caractérisé en ce que lesdites membranes sont traitées par un agent stabilisant au moins bifonctionnel susceptible de les rigidifier, ledit agent stabilisant pouvant être disloqué ou éliminé dans des conditions permettant de récupérer les membranes cellulaires ayant conservé l'essentiel de leurs propriétés biologiques et antigéniques.

La stabilisation obtenue par le procédé selon la présente invention permet non seulement de protéger les cellules contre une réaction de lyse liée à un choc osmotique, mais aussi de maintenir l'intégrité des antigènes de surface ainsi que des propriétés biologiques desdites cellules, membranes ou stromas. On entend par "propriétés biologiques", notamment la mobilité et la déformabilité des membranes cellulaires, ces propriétés étant nécessaires notamment aux réactions d'hémagglutination dans les divers systèmes de groupes sanguins.

Le procédé selon l'invention est particulièrement caractérisé en ce que la dislocation ou l'élimination de l'agent stabilisant, ou déprotection des membranes cellulaires, permet de retrouver ces dernières intactes. On entend par "membranes cellulaires intactes" des membranes ayant conservé l'essentiel de leurs propriétés biologiques et antigéniques.

L'agent stabilisant est, de préférence, selon la présente invention un réactif bifonctionnel du type $F_1$-X-$F_2$ dans lequel $F_1$ et $F_2$ peuvent être identiques ou différents et sont choisis parmi des groupes susceptibles de réagir avec les protéines ou les glucides de la membrane et/ou de la cellule, ou avec les fonctions -$NH_2$ ou -SH desdites protéines, et dans lesquels X peut être une fonction clivable ou un radical comportant une fonction clivable ou une liaison entre $F_1$ et $F_2$ qui peut être clivable ou non clivable.

Dans un premier mode de mise en oeuvre du procédé selon l'invention, la fonction X ou la liaison X est clivable par utilisation d'un réactif spécifique, chimique ou biologique, acide, enzyme ou autre.

Dans un second mode de mise en oeuvre du procédé, c'est l'une des fonctions $F_1$ ou $F_2$ ou bien les deux fonctions qui peuvent être clivées dans des conditions du même type que précédemment.

Dans ces deux cas, l'agent stabilisant pourra être disloqué.

Dans un troisième mode de mise en oeuvre du procédé, le radical bifonctionnel sera éliminé, c'est-à-dire que le clivage s'effectuera au-delà des fonctions $F_1$ et $F_2$ sur les sites membranaires ; ainsi, lorsque $F_1$-X-$F_2$ est fixé sur un peptide membranaire, l'utilisation d'une protéase permettra, par action sur le peptide, d'éliminer $F_1$-X-$F_2$.

Dans chacun de ces cas, les réactifs mis en oeuvre et les conditions réactionnelles devront être compatibles avec la conservation, pour l'essentiel, des propriétés biologiques et antigéniques des membranes cellulaires, en particulier du point de vue de la réactivité antigénique autologue.

$F_1$ et $F_2$ sont, de préférence, selon la présente invention, choisis dans le groupe des nombreux réactifs de réticulation utilisés pour le couplage ou la réticulation des composés antigéniques ou macromoléculaires. Ce groupe comprend les imidoesters, les esters de N-hydroxysuccinimide, les halogénures de nitroaryl, les carbodiimides, les cyanates, les aldéhydes, les maléimides, les thiophthalimides, les disulfures de pyridyle, les halogènes activés, les nitrènes ou les carbènes précurseurs.

Certaines parmi ces fonctions représentant $F_1$ et/ou $F_2$ se fixent de préférence sur une fonction aminoréactive spécifique de protéines membranaires du type R-$NH_2$ où R représente le résidu protéique.

Il en est ainsi, en particulier, pour les imidoesters, les esters de N-hydroxysuccinimide, les halogénures de nitroaryl carbodiimides, les cyanates et les aldéhydes. Les réactions spécifiques suivantes permettent la stabilisation selon la présente invention :

1) Imidoester

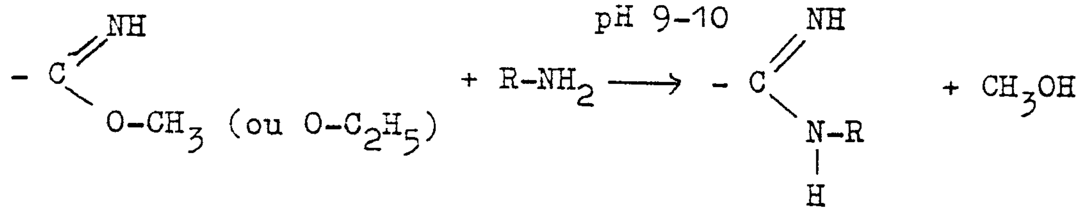


2) N-hydroxysuccinimide (NHS) ester

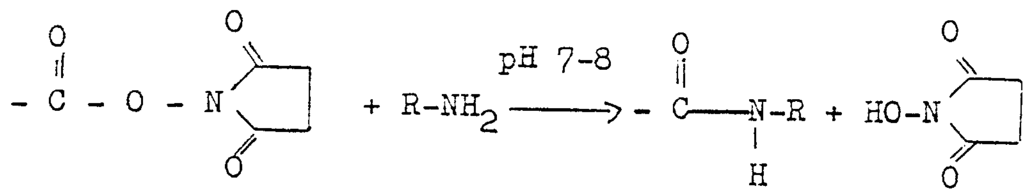


3) Halogénures de nitroaryl

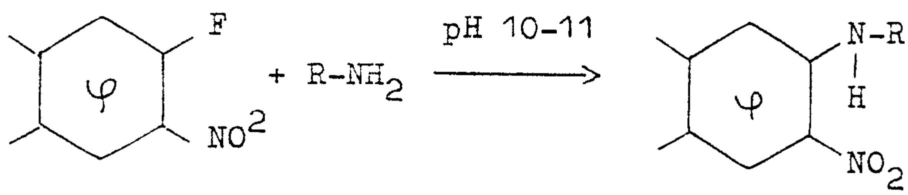

4) Carbodiimides

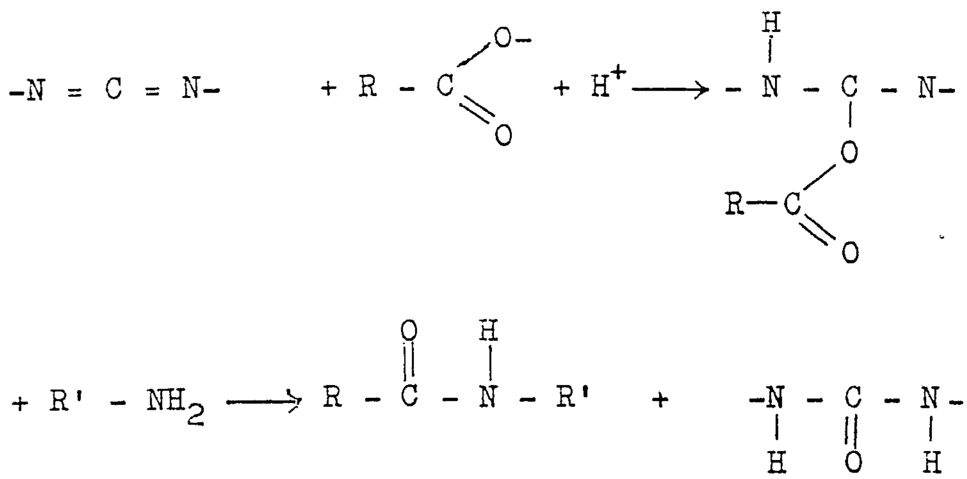

(R et R' représentent des résidus protéiques)

5) Cyanates

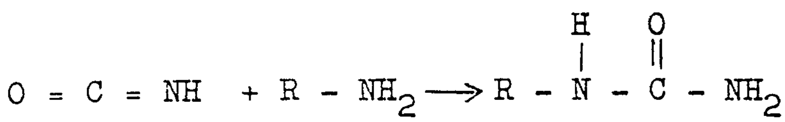 (carbamylation)

6) Aldéhydes

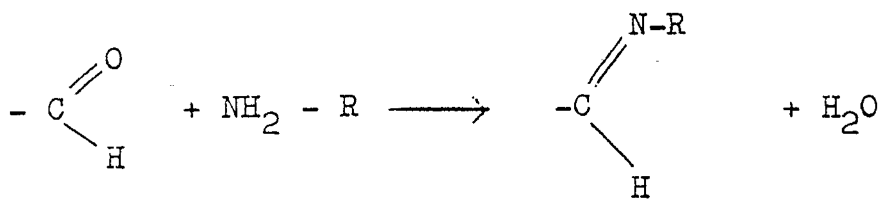

D'autres parmi ces fonctions représentant $F_1$ et/ou $F_2$ se fixent de préférence sur une fonction sulfhydryl réactive spécifique de protéines membranaires, du type SH-R, où R représente le résidu protéique. Il en est ainsi, en particulier, pour les maléimides, les thiophthalimides, les disulfures de pyridyle et les halogènes activés. Les réactions spécifiques suivantes permettent la réaction selon la présente invention :

1) Maléimides

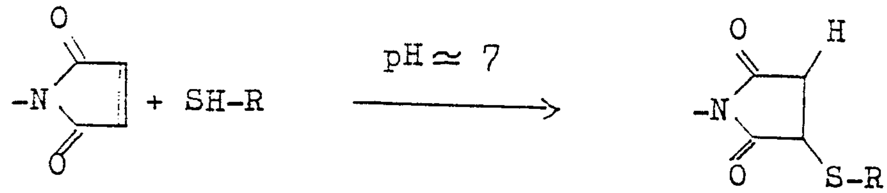


2) Thiophtalimides

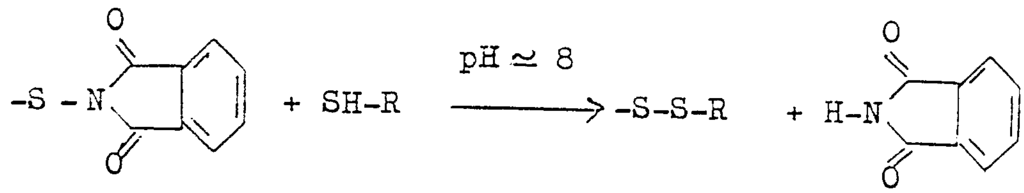


3) Disulfure de pyridyl

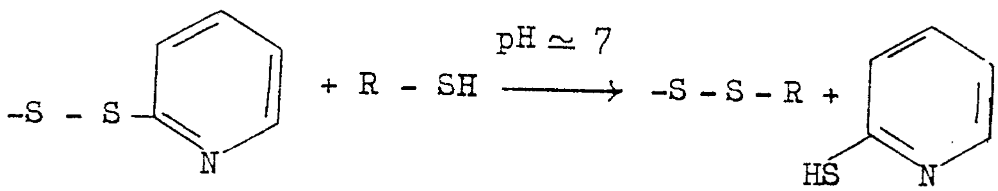


4) Halogènes activés

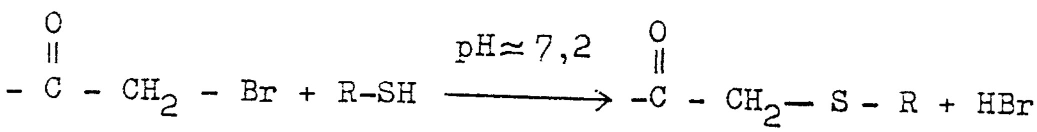


Enfin, certaines parmi ces fonctions représentant $F_1$ et/ou $F_2$ se fixent de préférence sur des fonctions réactives non spécifiques de protéines. Il en est ainsi pour les nitrènes précurseurs et les carbènes précurseurs.

Les réactions suivantes permettent la stabilisation des membranes cellulaires selon la présente invention, et ce grâce à une photoactivation :

1) Nitrènes précurseurs

$$R - N_3 \xrightarrow{h\gamma} R - N + N_2 \text{ (activation)}$$

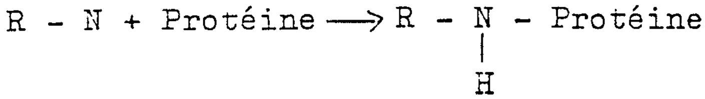

2) Carbènes précurseurs

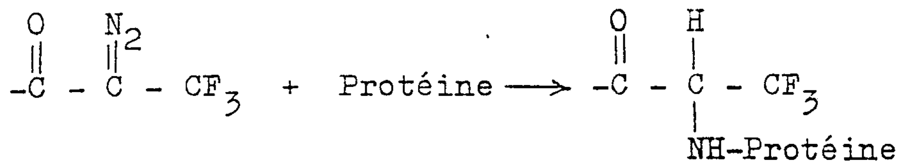

Les réactifs $F_1$-X-$F_2$ peuvent également, selon la présente invention, être obtenus en deux étapes par réaction d'un réactif de type:

$2F_1$-$R_1$ + $R_2$-X-$R_2$ → $F_1$-X-$F_1$

ou bien

$F_1$-$R_1$ + $F_2$-$R_3$ + $R_2$-X-$R_4$ → $F_1$-X-$F_2$

dans lesquels $R_1$ et R ainsi que $R_3$ et $R_4$ sont capables de réagir ensemble.

Suivant les conditions de réaction de $R_1/R_2$ $R_3/R_4$ et des groupements $F_1$ et $F_2$ sur les groupements R-$NH_2$, R-SH ou tout autre groupement réactif présent sur les protéines ou glucides de la membrane et/ou de la cellule, il sera possible de procéder de trois manières différentes pour aboutir à la stabilisation de la membrane :

1) - Dans un premier temps, synthèse, ou formation en milieu aqueux, du réactif $F_1$-X-$F_1$ extratemporanément par réaction du réactif $F_1$-$R_1$ sur le composé $R_2$-X-$R_2$.

- Dans un deuxième temps, stabilisation de la membrane par réaction du réactif $F_1$-X-$F_1$ obtenu précédemment avec les groupements réactifs présents sur les protéines ou glucides de la membrane et/ou de la cellule.

2) - Mélange simultané des trois systèmes réactifs $F_1$-$R_1$,$R_2$-X-$R_2$ et de la suspension cellulaire à stabiliser si les conditions de réaction de l'ensemble des trois systèmes le permettent.

3) - On peut également envisager d'activer les fonctions réactives présentes sur la membrane ou la cellule en faisant réagir d'abord $F_1$-$R_1$ puis ensuite faire réagir $R_2$-X-$R_2$. Bien entendu, les réactions seraient du même type avec un réactif dissymétrique $F_1$-X-$F_2$

L'avantage des réactifs $F_1$-X-$F_2$ obtenus selon ces procédés par rapport aux réactifs bifonctionnels du type $F_1$-X-$F_2$ décrits précédemment tient au plus large éventail de choix qu'ils procurent et au fait qu'ils permettent d'éviter la synthèse chimique de réactifs instables. Ainsi, il sera possible d'utiliser des réactifs $F_1$-X-$F_1$ obtenus à partir de réactifs $R_2$-X-$R_2$ comme les diacides ou dithioacides et de réactifs $F_1$-$R_1$ pouvant réagir à la fois sur des fonctions COOH des diacides et sur les groupements réactifs des protéines ou glucides de la membrane et/ou de la cellule comme les carbodiimides, l'EEDQ (N-éthoxycarbonyl-2-éthoxy-1,2-dihydroquinoline) ou le réactif de Woodward (N-éthyl-5-phénylisoxazolium-3'-sulfonate).

Outre les fonctions $F_1$ et $F_2$ des réactifs du type $F_1$-X-$F_2$, les réactifs selon la présente invention comprennent X qui peut être une fonction clivable, ou de façon plus générale un radical comportant une fonction clivable, et qui dans ce cas est de préférence choisi parmi les esters, amidines, disulfures et les fonctions -N=N- ou 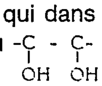

X peut être une fonction clivable et ce, selon différents procédés adaptés à sa nature. ainsi, le clivage peut être obtenu :

1) par réduction :

R-S-S-R' (pont disulfure) → R - SH + R' - SH

2) Par action du dithionite de sodium ($Na_2S_2O_6$) R - N = N - R' → R - $NH_2$ + R' - $NH_2$

3) Par oxydation périodique :

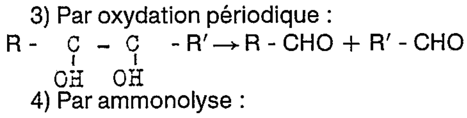

4) Par ammonolyse :

$R-\overset{NH}{\overset{\|}{C}}-NH-R'$ (amidine) $\rightarrow R'-NH_2 + R-\overset{NH}{\overset{\|}{C}}-NH_2$

5) Par action d'une base :

$R-NH-CH_2-OCO-CH_2-R' \rightarrow R-NHCH_2OH + R'CH_2COOH$

D'autre part, X peut représenter simplement une liaison clivable ou non entre $F_1$ et $F_2$, c'est le cas lorsque les fonctions $F_1$ et $F_2$ peuvent, après couplage, être directement clivées de leur site de réaction ou bien $F_1$ et $F_2$ étant clivés au niveau de leur liaison. Dans tous les cas le clivage selon l'invention ne peut être mis en oeuvre que dans des conditions permettant de récupérer des membranes cellulaires ayant conservé l'essentiel de leurs propriétés biologiques ou antigéniques.

Dans le cas où le réactif bifonctionnel du type $F_1$-X-$F_2$ ne possède pas de fonction à caractère clivable, l'élimination de l'agent stabilisant ou la déprotection des membranes cellulaires peut être effectuée par action d'une ou plusieurs protéases, comme cela est connu dans un laboratoire d'immunohématologie pour d'autres applications, de préférence choisies parmi celles utilisées en immunohématologie comme la broméline, la trypsine ou la papaïne, dans des conditions permettant de récupérer des membranes cellulaires ayant conservé l'essentiel de leurs propriétés biologiques et antigéniques.

Les réactifs $F_1$-X-$F_2$ ne sont aucunement limités à ceux exemplifiés ci-dessus. En effet, la condition impérative de leur utilisation est qu'ils permettent une rigidification et une déprotection des membranes cellulaires dans des conditions préservant les propriétés biologiques ou antigéniques de ces dernières. Ainsi, les conditions de mise en oeuvre de ces réactifs sont tout aussi importantes que le choix proprement dit des réactifs. On sait, par exemple, que des réactions par voie enzymatique sont modérées et permettent de ménager l'intégrité de la cellule ou de la membrane.

La présente invention s'étend donc aux réactifs stabilisant bifonctionnels susceptibles de rigidifier les membranes cellulaires par voie enzymatique. C'est ainsi que les résidus amine d'une diamine clivable (telle que $H_2N-CH_2-CH_2S-SCH_2-CH_2-NH_2$) peuvent être transférés à l'aide de la transglutaminase sur des résidus glutamines de protéines cellulaires.

La présente invention s'étend aussi aux réactifs stabilisants dislocables dans des conditions enzymatiques, tels que ceux possédant une fonction X qui peut être un polypeptide, un phosphate ou un ester, choisi pour pouvoir être clivé par voie enzymatique.

Le procédé selon la présente invention peut être utilisé pour la stabilisation de nombreuses cellules, membranes cellulaires ou stromas, mais on l'appliquera de préférence aux cellules sanguines et/ou aux cellules d'un organe hématopoïétique, telles que les érythrocytes, leurs stromas ou leur membrane, les lymphocytes, les plaquettes ou les granulocytes. Ces cellules, membranes ou stromas, une fois stabilisés, peuvent être utilisés en milieu liquide pour constituer des panels de détection des antigènes autologues pour la transfusion sanguine. Au surplus, la protection induite par la réticulation de la membrane permet d'envisager une conservation par congélation ou lyophilisation en mettant les cellules, membranes ou stromas, de façon transitoire, à l'abri d'un choc osmotique.

L'avantage surprenant, obtenu grâce au procédé selon l'invention, est le blocage réversible de la réactivité antigénique des cellules, notamment des globules rouges, traitées avec les réactifs $F_1$-X-$F_2$ mentionnés ci-dessus.

C'est pourquoi, les cellules, stromas ou membranes ainsi traités peuvent être conservés, congelés et même lyophilisés tout en gardant intactes leurs propriétés antigéniques après déprotection.

Le procédé selon la présente invention pourra également s'appliquer à des cellules à usage thérapeutique potentiel ; il suffira pour ce faire d'utiliser des réactifs $F_1$-X-$F_2$ biocompatibles. On pourra recourir, de préférence, dans ce cas à des réactifs autorisant une rigidification ou un clivage par voie enzymatique (protéases, estérases, phosphatases plasmatiques) afin s'obtenir des cellules déprotégées, biologiquement intègres et compatibles.

La présente invention s'étend aux diverses utilisations des cellules, stromas ou membranes traités par le procédé tel que décrit ci-dessus et, notamment, à la préparation de réactifs à l'aide de ces cellules, stromas ou membranes.

La présente invention sera mieux comprise à la lecture des exemples suivants relatifs à la lyophilisation des globules rouges.

EXEMPLE I

L'exemple ci-après décrit, en effet, en détail les résultats obtenus en faisant agir sur les érythrocytes le diméthyl-3, 3'-dithiobis proprionimidate (DTBP) qui est l'un des réactifs les plus représentatifs des familles décrites ci-dessus.

Le DTBP répond à la formule suivante :

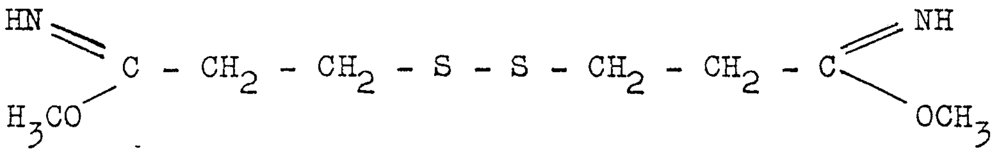

et la réaction de rigidification s'effectue sur les fonctions $R_1$-$NH_2$ et $R_2$-$NH_2$ spécifiques des protéines érythrocytaires ($R_1$ et $R_2$ représentent les résidus protéiques) de la façon suivante :

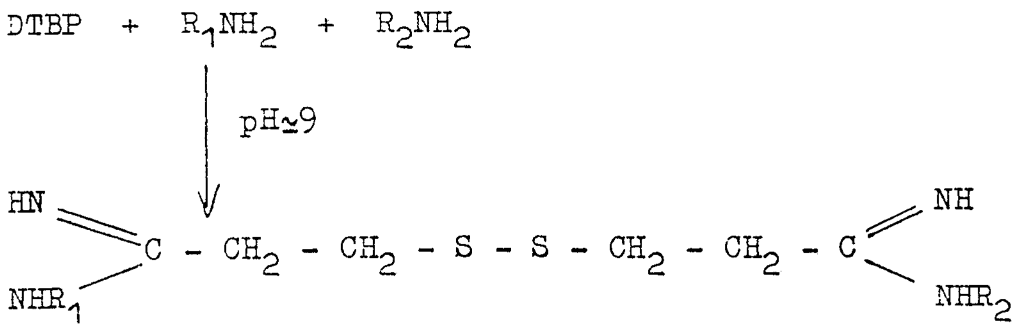


La figure 1 représente la fragilité osmotique résiduelle des globules rouges. Sur cette figure :

représente le témoin globules rouges non traités,

représente les globules rouges traités au DTBP (8 mM)

représente les globules rouges traités au DTBP puis réduits et alkylés (mercapto-éthanol plus iodoacétamide 12,8 mM).

Exemple de procédé

Stabilisation de globules rouges d'origine humaine par le diméthyl 3,3′ dithiobispropionimidate (DTBP), réactif dislocable :

A - Les réactifs et solutions

1) Globules rouges

Fournis par le Centre de Transfusion Sanguine (CTS) de Tours, recueillis sur CPD ou CPD-SAG mannitol (concentrés globulaires en général).

2) Les réactifs

- Le DTBP, fournisseur : Réf.: SIGMA D 2388 (PM : 309,8)
- Le 2-mercapto-éthanol, Prolabo
- L'iodoacétamide (Fluka)
- Les sérums test (CNTS d'Orsay)

3) Les solutions

- NaCl 9 ‰ en eau distillée, 300 mosm/kg d'eau
- Tampon Tris-HCl 0,2 M, pH 9,2, réajuster à 300 mosm/kg avec NaCl
- Tampon phosphate 0,01 M, pH 7,1, réajuster à 300 mosm/kg avec NaCl (P.B.S.).

B - Réaction de rigidification

1) Préparation des globules rouges

Le concentré de globules rouges est décanté puis lavé 3 fois en NaCl 9 ‰ pour être finalement resuspendu

en NaCl 9 ‰ à un hématocrite de 85 % (± 2 %).

2) Rigidification

Pour 1 ml de culot de globules rouges traités à 6,5 mM de DTBP.

Dans un tube pouvant contenir 10 ml, on ajoute 8 ml de tampon Tris HCl (cf solution) préalablement ramené à température ambiante aux 18,1 mg de DTBP (pesé auparavant directement dans le tube). L'ensemble est agité (au vortex) quelques secondes (dissolution immédiate).

Aux 8 ml, on ajoute immédiatement 1 ml du culot à traiter (durée de vie du réactif très brève) (le culot est ramené également à température ambiamte).

Le tube est incubé pendant 30 minutes à température ambiante sous agitation lente (par retournement).

Au bout de 30 minutes, le tube est centrifugé 10 minutes à +4°C à 2400 rpm, décanté, puis le culot est lavé 2 fois en PBS (par centrifugation et décantation successives, 10 min à 2400 rpm).

Le culot étant "pris en masse" à la fin de chaque centrifugation, il est nécessaire de le remettre en suspension à l'aide d'une pipette pasteur.

Une légère hémolyse apparaît en général après le premier lavage en PBS.

C - Réaction de réduction-alkylation

1) Préparation du culot traité au DTBP

Au dernier lavage, on obtient 1 ml de culot à environ 80 % Hc.

A ces 1 ml, on ajoute 1 ml de PBS (le tout est homogénéisé à l'aide d'une pipette pasteur et d'un vortex) (Hc environ 40 %).

2) Préparation des solutions de réduction et d'alkylation

. Solution de réduction :

On prépare une solution à 5 % (v/v) en mercaptoéthanol dans du PBS (1 ml de mercaptoéthanol pur pour 19 ml de PBS).

. Solution d'alkylation :

On prépare une solution de 8,74 % (p/v) en iodoacétamide dans du PBS (87,4 mg de iodoacétamide pour 1 ml de PBS).

3) Réduction-alkylation d'un culot de globules rouges au DTBP

A 1 ml de PBS on ajoute :
- 40 µl de mercaptoéthanol à 5 % puis agitation vortex,
- 50 µl d'iodoacétamide à 8,74 % puis agitation vortex,
- puis, immédiatement, on ajoute 1 ml de culot traité au DTBP (ramené à 40 % d'Hc avec PBS) (concentration finale en mercaptoéthanol : 12,8 mM ou 0,1 % - concentration finale en iodoacétamide : 12,8 mM).

L'ensemble est incubé 10 minutes à température ambiante sous agitation par retournement du tube.

Au bout de 10 minutes, le tube contenant 2 ml est complété à 5 ml avec NaCl 9 ‰ puis centrifugé à 2400 rpm pendant 10 minutes.

Le culot obtenu est ensuite lavé (1 à 2 fois) en NaCl 9 ‰.

Les différentes etapes de ce procédé sont résumées dans le schéma type suivant :

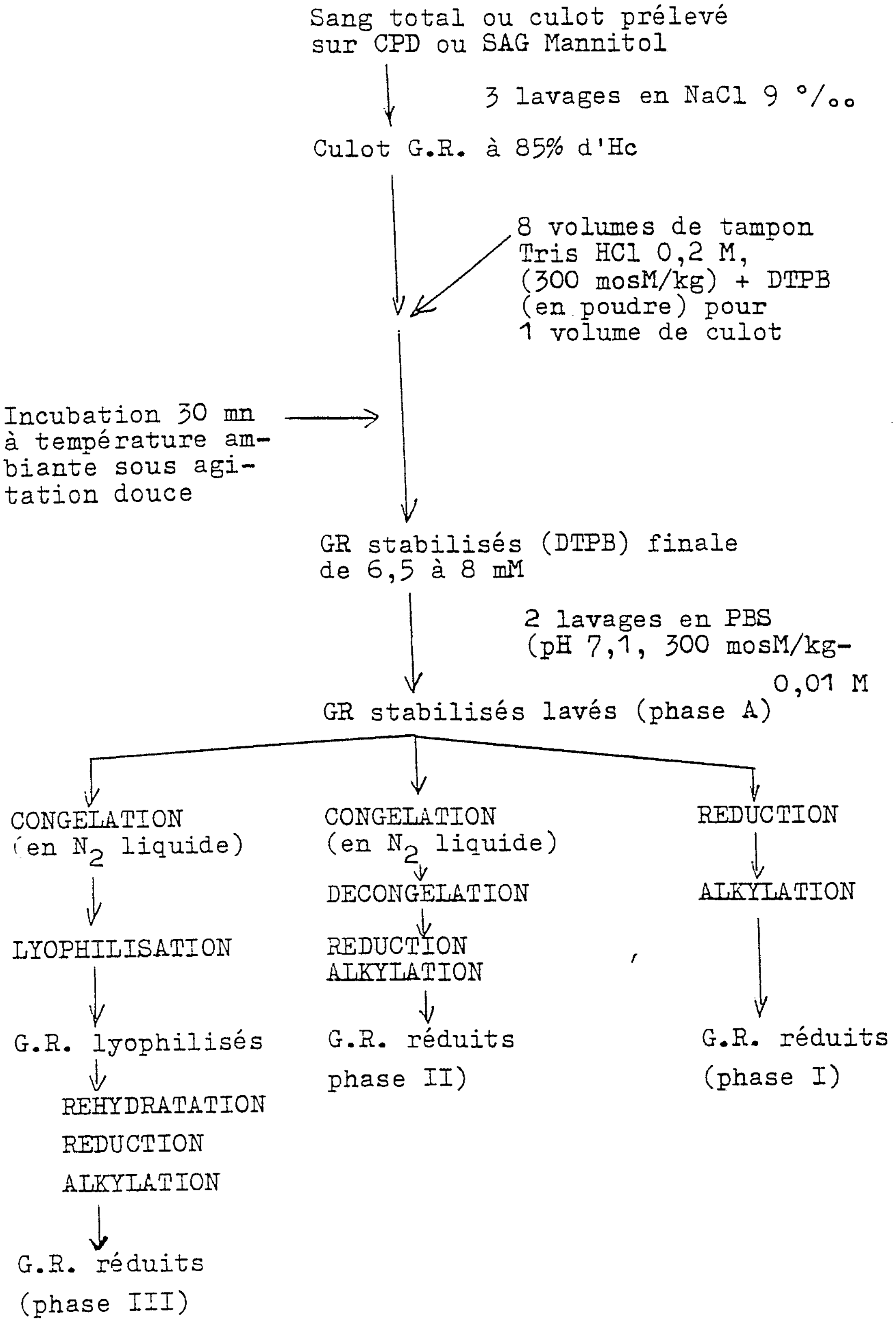
Sang total ou culot prélevé sur CPD ou SAG Mannitol
3 lavages en NaCl 9 °/oo
Culot G.R. à 85% d'Hc
8 volumes de tampon Tris HCl 0,2 M, (300 mosM/kg) + DTPB (en poudre) pour 1 volume de culot
Incubation 30 mn à température ambiante sous agitation douce
GR stabilisés (DTPB) finale de 6,5 à 8 mM
2 lavages en PBS (pH 7,1, 300 mosM/kg-0,01 M
GR stabilisés lavés (phase A)
CONGELATION (en $N_2$ liquide)
LYOPHILISATION
G.R. lyophilisés
REHYDRATATION
REDUCTION
ALKYLATION
G.R. réduits (phase III)
CONGELATION (en $N_2$ liquide)
DECONGELATION
REDUCTION
ALKYLATION
G.R. réduits phase II)
REDUCTION
ALKYLATION
G.R. réduits (phase I)

Principaux résultats

1) Fragilité osmotique des globules rouges ainsi traités

Les courbes de fragilité osmotique obtenues par détermination du pourcentage d'hémolyse pour une gamme de pressions osmotiques donnée (figure 1) montrent que :
- Lorsque le globule rouge est uniquement traité par action du DTBP (phase A sur le schéma) sa résistance à la lyse en milieu hypotonique est très accrue (peu ou pas de lyse en eau distillée). A ce stade, les globules rouges peuvent être congelés en azote liquide puis décongelés en l'absence de cryoprotecteurs (peu d'hémolyse). Il en est de même pour la lyophilisation.
- Lorsque le globule rouge est "réduit-alkylé" (phase I sur le schéma) après traitement au DTBP, la courbe de fragilité osmotique reprend une allure pratiquement normale par rapport au témoin avec un déplacement d'environ 30 mosm/kg à droite pour le 50 % d'hémolyse.

2) Réactivité antigénique des globules rouges ainsi traités

Lorsque le globule rouge est traité au DTBP (phase A) à une concentration finale en DTBP supérieure ou égale à 5 mM, les antigènes A, B et du groupe rhésus (D, C, $\bar{c}$, E, $\bar{e}$) ont pratiquement totalement perdu leur réactivité (très faible pour le A et B).

Lorsque le globule rouge est "réduit-alkylé" (phase I) après traitement au DTBP et conservé en milieu liquide, les résultats obtenus pour les antigènes testés sont les suivants (tableau I) :

TABLEAU I

| Antigène Spécificité | Globule rouge témoin | Globule rouge traité au DTBP réduit-alkylé | Technique de groupage |
|---|---|---|---|
| A | +++ | +++ | sur plaque d'opaline |
| B | +++ | +++ | sur plaque d'opaline |
| D | +++ | +++ | sur plaque d'opaline |
| C | ++ | ++ | sur plaque d'opaline |
| $\bar{c}$ | ++ | ++ | sur plaque d'opaline |
| E | ++ | ++ | sur plaque d'opaline |
| $\bar{e}$ | ++ | ++ | sur plaque d'opaline |
| Fya | + | ++ | Coombs BFI |
| Fyb | + | ++ | Coombs BFI |
| JKa | ++ | ++ | Coombs BFI |
| JKb | ++ | ++ | Coombs BFI |
| M | +++ | +++ | Milieu salin |
| N | +++ | +++ | Milieu salin |
| $P_1$ | +++ | +++ | Coombs BFI |
| S | +++ | +++ | Coombs BFI |
| s | +++ | +++ | Coombs BFI |
| a | ++ | ++ | Coombs indirect |

(Les croix représentent l'intensité de la réaction d'agglutination). Les globules rouges témoins ou traités au DTBP puis "réduit-alkylés" ont une réaction négative en présence de sérum AB en milieu salin, en Coombs normal, Coombs BFI et en papaïne, ce qui traduit une absence d'antigènes de polyagglutinabilité.

EXEMPLE II

L'exemple ci-après décrit les résultats obtenus en faisant agir sur les érythrocytes un mélange réactif préparé extratemporanément par réaction du 1,5-difluoro-2,4-dinitrobenzène (DFDNB) sur le glutathion oxydé (GSSG).

Le DFDNB répond à la formule suivante :

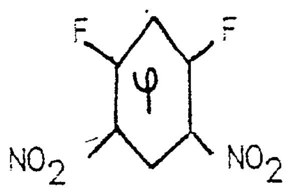

Le GSSG répond à la formule suivante :

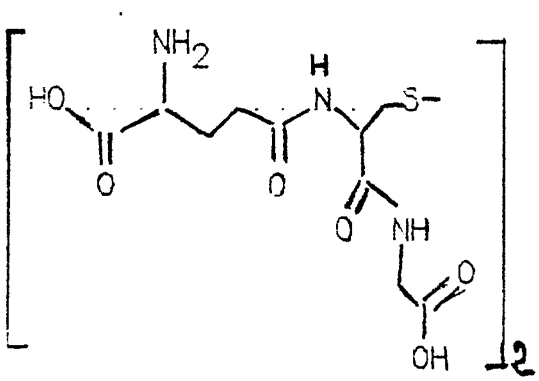

représentée schématiquement par :

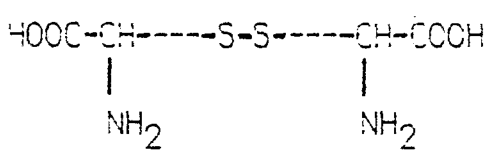

Préparation extratemporanée du réactif bifonctionnel clivable par mélange du DFDNB et du GSSG :

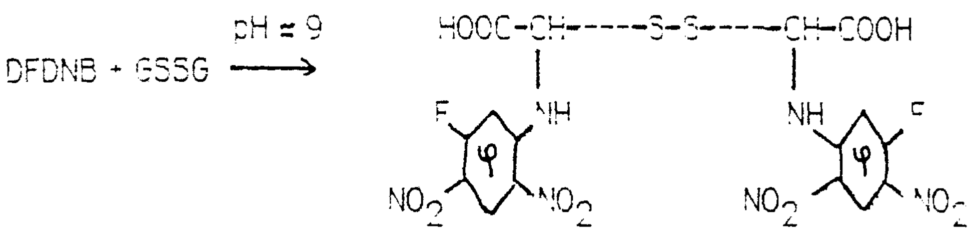


La réaction de rigidification s'effectue sur les fonctions $P_1$-$NH_2$ et $P_2$-$NH_2$ spécifiques des protéines érythrocytaires ($P_1$ et $P_2$ représentant les résidus protéiques) de la manière suivante :

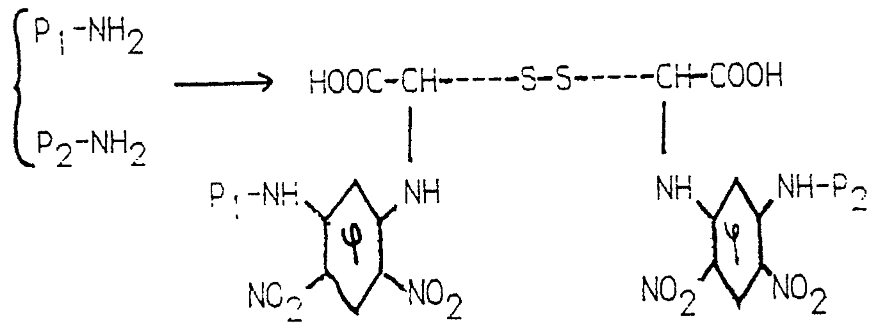

Exemple de procédé

Stabilisation de globules rouges d'origine humaine par une solution obtenue à partir d'un mélange de réactifs préparé extratemporanément par réaction du 1,5-difluoro-2,4-dinitrobenzène (DFDNB) sur le glutathion oxydé (GSSG), réactif dislocable

A - Les réactifs et solutions

1) les globules rouges

Fournis par le Centre de Transfusion Sanguine (CTS) de Tours, recueillis sur CPD ou CPD-SAG mannitol (concentrés globulaires en général).

2) Les réactifs

- Le DFDNB fournisseur Pearce réf. 21524
- Le GSSG fournisseur Fluka réf. 49740
- Le 2-mercapto-éthanol Prolabo
- L'iodoacétamide Fluka
- Les sérums test CNTS d'Orsay

3) Les Solutions

- NaCl 9‰ en eau distillée, 300 mosm/kg d'eau
- Tampon phosphate 0,1 M, pH 8,5, réajuster à 300 mosm avec NaCl
- Tampons phosphates 0,1 M et 0,01 M, pH 7,1, réajuster à 300 mosm avec NaCl.

B - Réactions de rigidification

1) Préparation des globules rouges

Le concentré de globules rouges est décanté puis lavé 3 fois en NaCl 9 ‰ pour être finalement resuspendu en NaCl 9 ‰ à un hématocrite de 85%.

2) Rigidification

a) Préparation extratemporanée de la solution de réactif bifonctionnel clivable de couplage.

A une solution de DFDNB comprise entre 100 mM et 500 mM en méthanol pur, un volume égal de solution de GSSG comprise entre 50 mM et 250 mM en PBS 0,1 M ; pH 8,5 est ajouté, le rapport molaire de DFDNB/GSSG de la solution avoisinant 2. La solution obtenue est incubée pendant environ 30 minutes à température ambiante sous agitation douce.

b) Stabilisation des globules rouges pour 1 ml de culot

Dans un tube pouvant contenir 10 ml, on ajoute 8 ml de tampon PBS 0,1 M ; pH 8,5 à 1 ml de culot de globules rouges ramené à environ 80% d'hématocrite 100 à 300 µl de la solution de réactif préalablement incubée sont alors ajoutés. L'ensemble est ensuite incubé entre 30 minutes et 2 heures à température ambiante et sous agitation.

Au bout de cette incubation, le tube est centrifugé 10 minutes à + 4° C à 2000 rpm, décanté puis le culot est lavé 2 fois en PBS 0,1 M ; pH 7,1 et une fois en PBS 0,01 M ; pH 7,1 (par centrifugation et décantation successives).

c) Réaction de réduction-alkylation

1) Préparation du culot traité au mélange DFDNB-GSSG préincubé

Au dernier lavage, on obtient 1 ml de culot à 80% d'Hc. que l'on ramène à 40% d'Hc. avec du PBS 0,01 M ; pH 7, 1.

2) Préparation des solutions de réduction-alkylation

. Solution de réduction

On prépare une solution à 5% (v/v) en mercaptoéthanol dans du PBS (1 ml de marcaptoéthanol pur pour 19 ml de PBS).

. Sodium d'alkylation

On prépare une solution à 8,74% (en p/v) en iodacétamide dans du PBS (87.4 mg de iodoacétamide pour 1 ml de PBS).

3) Réduction-alkylation d'un culot de globules rouges traité au mélange DFDNB-GSSG

A 1 ml de PBS on ajoute :

- 40 µl de mercaptoéthanol à 5% puis Agitation vortex
- 50 µl de iodoacétamide à 8,74% puis Agitation vortex

Puis immédiatement, on ajoute 1 ml de culot traité au mélange DFDNB-GSSG (ramené à 40% d'Hc avec PBS) (concentration finale en mercaptoéthanol : 12,8 mM ou 0,1% - Concentration finale en iodoacétamide : 12,8 mM).

L'ensemble est incubé 10 min à température ambiante sous agitation par retournement du tube.

Au bout de 10 min, le tube contenant 2 ml est complété à 5 ml avec NaCl 9 ‰ puis centrifugé à 2 000 rpm pendant 10 min.

Le culot obtenu est ensuite lavé (1 à 2 fois) en NaCl 9 ‰.

<u>Principaux résultats</u>

1) Fragilité osmotique des globules rouges ainsi traités

Lorsque le globule rouge est uniquement traité au mélange DFDNB-GSSG, sa résistance à la lyse hypotonique est très accrue (peu ou pas de lyse en eau distillée). A ce stade du procédé les globules rouges peuvent être congelés puis décongelés en l'absence de cryoprotecteurs (peu d'hémolyse). Il en est de même pour la lyophilisation.

2) Réactive antigénique des globules rouges ainsi traités

Lorsque le globule rouge est traité uniquement au mélange DFDNB-GSSG pour les concentrations données précédemment, la réactivité antigénique des antigénes de groupes sanguins comme l'antigéne A est très atténuée.

Lorsque le globule rouge est "réduit-alkylé" après rigidification ou après rigidification et congélation-décongélation sand cryoprotecteur, la réactivité antigénique de l'antigéne A réapparait normalement (cf tableau suivant).

| Sérums tests | Anti A | Anti B | Anti (A+B) |
|---|---|---|---|
| Globule rouge témoin non traité | +++ | - | +++ |
| Globule rouge traité au mélange DFDNB-GSSG | + | - | - |
| Globule rouge traité puis réduit-alkylé | +++ | - | +++ |
| Globule rouge traité, congelé décongelé puis réduit-alkylé | +++ | - | +++ |

Les croix représentent l'intensité de la réaction d'agglutination suivant les normes adoptées en immunohématologie.

EXEMPLE III

L'exemple ci-après décrit les résultats obtenus en faisant agir le dimethyl adipimidate (DMA), réactif non dislocable, suivi de l'action d'une protéase (la broméline) sur des érythrocytes d'origine humaine.

Le DMA répond à la formule suivante :

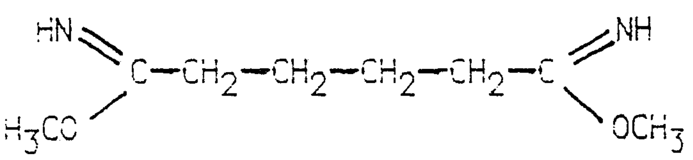

La réaction de rigidification s'effectue suivant le même schéma que pour le DTBP (cf EXEMPLE I) dont la formule est identique hormis le groupement disulfure.

Exemple de procédé

Stabilisation de globules rouges humains par le dimethyl adipimidate (DMA), réactif non dislocable.

A - Les réactifs et solutions

1) Globules rouges

Fournis par le Centre de Transfusion Sanguine (CTS) de Tours, recueillis sur CPD ou CPD-SAG mannitol (concentrés globulaires en général).

2) Les réactifs
- le DMA : Fournisseur Pearceref. 20664
- la Broméline : Fournisseur Serva ref. 15250
- les sérums test CNTS d'Orsay.

3) Les solutions
- NaCl 9 0/00 en eau distillée, 300 mosm/kg d'eau
- Tampon Tris-HCl 0,2 M ; pH 9,2 ; réajuster à 300 mosm/kg avec NaCl
- Tampon phosphate 0,01 M ; pH 7,1 ; réajuster à 300 mosm/kg avec NaCl (P.B.S.).
- Tampon basse force ionique standard (BFI).

B - Réactions de rigidification

1) Préparation des globules rouges

Le concentré de globules rouges est décanté puis lavé 3 fois en NaCl 9 0/00 pour être finalement resuspendu en NaCl 9 0/00 à un hématocrite de 85%.

Rigidification

- Pour 1 ml de culot de globule rouge traité à 6,5 mM de DMA (concentration pouvant varier entre 3 mM et 10 mM suivant l'activité du lot de DMA utilisé).

Dans un tube pouvant contenir 10 ml, on ajoute 8 ml de Tampon Tris HCl (cf solution) préalablement ramené à température ambiante à la quantité de DMA désirée (pesée auparavant directement dans le tube). L'ensemble est agité (au vortex) quelques secondes (dissolution immédiate).

Aux 8 ml, on ajoute immédiatement 1 ml du culot à traiter (durée de vie du réactif très brève) (le culot est ramené également à température ambiante).

Le tube est incubé pendant 30 minutes à température ambiante sous agitation lente (par retournement).

Au bout de 30 minutes, le tube est centrifugé 10 min à +4° C à 2400 rpm, décanté puis le culot est lavé 2 fois en PBS (par centrifugation et décantation successives, 10 min à 2400 rpm).

Le culot étant "prise en masse" à fin la de chaque centrifugation, il est nécessaire de le remettre en suspension à l'aide d'une pipette pasteur.

Une légère hémolyse apparaît en général après le premier lavage en PBS.

C - Action de la broméline

1) Préparation du culot traité au DMA

Au dernier lavage, on obtient 1 ml de culot à environ 80 % Hc.

A ces 1 ml, on ajoute 1 ml de BFI (le tout est homogénéisé à l'aide d'une pipette pasteur et d'un vortex) (Hc environ 40 %).

2) Préparation de la solution de broméline

La solution de broméline est préparée le même jour.

A 1 litre de tampon BFI, on ajoute 0,72 g. de broméline. Après dissolution, la solution obtenue est filtrée sur papier Wathman puis conservée à +4°C, 24 heures au maximum avant utilisation.

3) Action de la broméline

A 0,5 ml de culot traité au DMA, on ajoute 4,5 ml de la solution de broméline. L'ensemble est incubé 15 minutes à température ambiante.

Après incubation, la suspension est centrifugée 5 minutes à 2000 rpm puis décantée. Le culot est ensuite lavé 2 fois en NaCl 9 0/00 par centrifugation-décantation.

Principaux résultats

1) Fragilité osmotique des globules rouges obtenus ainsi traités

Lorsque le globule rouge est uniquement traité au DMA, leurs caractéristiques concernant leur fragilité osmotique sont identiques à celles obtenues par le traitement au DTBP et énoncées précédemment :
- Résistance accrue à la lyse en milieu hypotonique

-Taux d'hémolyse faible après congélation-décongélation ou lyophilisation-réhydratation sans cryoprotecteur.

Lorsque les globules rouges sont traités au DMA puis soumis à l'action de la broméline, la résistance à la lyse en milieu hypotonique diminue mais reste encore très accrue.

2) Réactivité antigénique des globules rouges ainsi traités

Lorsque le globule rouge est traité uniquement au DMA, les antigénes A, B et du groupe Rhésus (D, C, $\bar{c}$, E, $\bar{e}$) ont pratiquement totalement perdu leur réactivité antigénique.

Lorsque les globules rouges sont soumis ensuite à l'action de la broméline les résultats obtenus pour les antigénes testés sont les suivants :

| Antigènes Spécificité | Glob. rouge témoin | Glob. rouge traité au DMA seul | Glob. rouge traité au DMA + bromél. |
|---|---|---|---|
| A | +++ | + | +++ |
| D | +++ | (+) | +++ |
| C | ++ | (+) | ++ |
| $\bar{c}$ | ++ | (+) | ++ |
| E | ++ | (+) | ++ |
| $\bar{e}$ | ++ | (+) | ++ |

Pour chaque type de globule rouge, l'agglutination en présence de sérum AB en milieu salin a été négative, la technique de groupage sur plaque d'opaline a été utilisé pour l'ensemble des spécificités.

Cryoprotection et lyophilisation pour les trois exemples précités.

Les globules rouges traités au DTBP du mélange DFDNB-GSSG et au DMA sont très résistants et peuvent être congelés directement eu eau distillée et décongelés dans les mêmes conditions (phase II sur le schéma). Le rendement de récupération après décongélation est pratiquement de 100%. Cependant, les conditions brutales de pression osmotique peuvent induire une destruction antigénique partielle ainsi que modifier les conditions de réduction alkylation ou d'action des protéases. Pour atténuer ces effets, il est souhaitable d'utiliser des cryoprotecteurs internes et/ou externes, en vue de récupérer la réactivité antigénique total après réduction et alkylation ou action des protéases.

Le rendement de la récupération est pratiquement de 100% (peu ou pas de lyse). La réactivité antigénique pour les antigènes A et B est récupérée après réduction et alkylation.

En ce qui concerne la lyophilisation, (phase III sur le schéma), la situation est identique : les globules stabilisés peuvent être lyophilisés directement. La recherche d'une récupération optimale des cellules et de leur réactivité antigénique met en jeu l'usage de lyoprotecteurs, de solutions de réhydration, de cinétique de déprotection pour éviter un choc osmotique excessif produisant des dommages irréversibles. La rigidification temporaire de la membrane permet de s'affranchir de la brutalité d'une réhydration directe conduisant à une dégradation immédiate et irréversible de la cellule. Les optimisations correspondantes sont en cours.

**Revendications**

1. Procédé de stabilisation des membranes cellulaires, en vue notamment de conserver les cellules, membranes ou stromas sous une forme protégeant l'essentiel de leurs antigènes de surface, caractérisé en ce que lesdites membranes sont traitées par un agent stabilisant au moins bifonctionnel susceptible de les rigidifier, ledit agent stabilisant pouvant être disloqué ou éliminé dans des conditions permettant de récupérer les membranes cellulaires ayant conservé l'essentiel de leurs propriétés biologiques et antigéniques.

2. Procédé selon la revendication 1, caractérisé en ce que l'agent stabilisant est un réactif du type

$F_1$-X-$F_2$ dans lequel $F_1$ et $F_2$ peuvent être identiques ou différents et sont choisis parmi des groupes chimiques susceptibles de réagir avec les protéines ou les glucides desdites membranes et/ou desdites cellules, ou avec les fonctions -$NH_2$ ou -SH desdites protéines et X est une fonction ou un radical comportant une fonction ou une liaison clivable ou non.

3. Procédé selon la revendication 2, caractérisé en ce que $F_1$ et $F_2$ sont choisis dans le groupe comprenant les imidoesters, les esters de N-hydroxy-succinimide, les halogénures de nitroaryl, les carbodiimides, les cyanates, les aldéhydes, les maléimides, les thiophthalimides, les disulfures de pyridyle, les halogènes activés, les nitrènes ou les carbènes précurseurs.

4. Procédé selon l'une des revendications 2 et 3, caractérisé en ce que le radical X est une fonction, un radical portant une fonction ou une liaison clivable dans des conditions permettant de récupérer les membranes cellulaires ayant conservé l'essentiel de leurs propriétés biologiques et antigéniques.

5. Procédé selon l'une des revendications 2 et 3, caractérisé en ce que X est une fonction ou comporte une fonction choisie dans le groupe comprenant les phosphates, polypeptides, esters, amidine, disulfures et les fonctions -N = N- ou $-\underset{OH}{\overset{|}{C}}-\underset{OH}{\overset{|}{C}}-$.

6. Procédé selon la revendication 2, caractérisé en ce que le réactif $F_1$-X-$F_2$ est préparé in situ par réaction d'un composé $F_1$-$R_1$ et/ou $F_2$-$F_3$ sur un composé de formule $R_2$-X-$R_4$ ou $R_2$-X-$R_2$, dans lesquelles $R_1$ et $R_2$ et $R_3$ et $R_4$ sont des fonctions réagissantes pour former une liaison.

7. Procédé selon l'une des revendications 1, 2 et 6, caractérisé en ce que l'agent stabilisant peut être éliminé par clivage sur les sites membranaires où se trouve fixé ledit agent stabilisant.

8. Procédé selon la revendication 7, caractérisé en ce que l'agent stabilisant peut être éliminé par clivage à l'aide de protéases.

9. Procédé selon l'une quelconque des revendications 1 à 8, caractérisé en ce que les cellules sont des cellules issues du sang et/ou des organe hématopoiétique.

10. Procédé selon la revendication 9, caractérisé en ce que les cellules sont des érythrocytes, leur stroma ou leur membrane.

11. Procédé selon la revendication 9, caractérisé en ce que les cellules sont des lymphocytes.

12. Procédé selon la revendication 9, caractérisé en ce que les cellules sont des granulocytes.

13. Procédé selon la revendication 9, caractérisé en ce que les cellules sont des plaquettes.

14. Procédé selon l'une quelconque des revendications 1 à 13, caractérisé en ce que les cellules, stromas ou membranes ont un potentiel thérapeutique et/ou antigénique.

15. Application du procédé selon l'une quelconque des revendications 1 à 14 à la congélation des cellules, leur stroma ou membrane.

16. Application du procédé selon l'une quelconque des revendications 1 à 15 à la lyophilisation des cellules, leur stroma ou membrane.

17. Application du procédé selon l'une quelconque des revendications 1 à 16 à la conservation des cellules, stromas ou membranes.

0297958

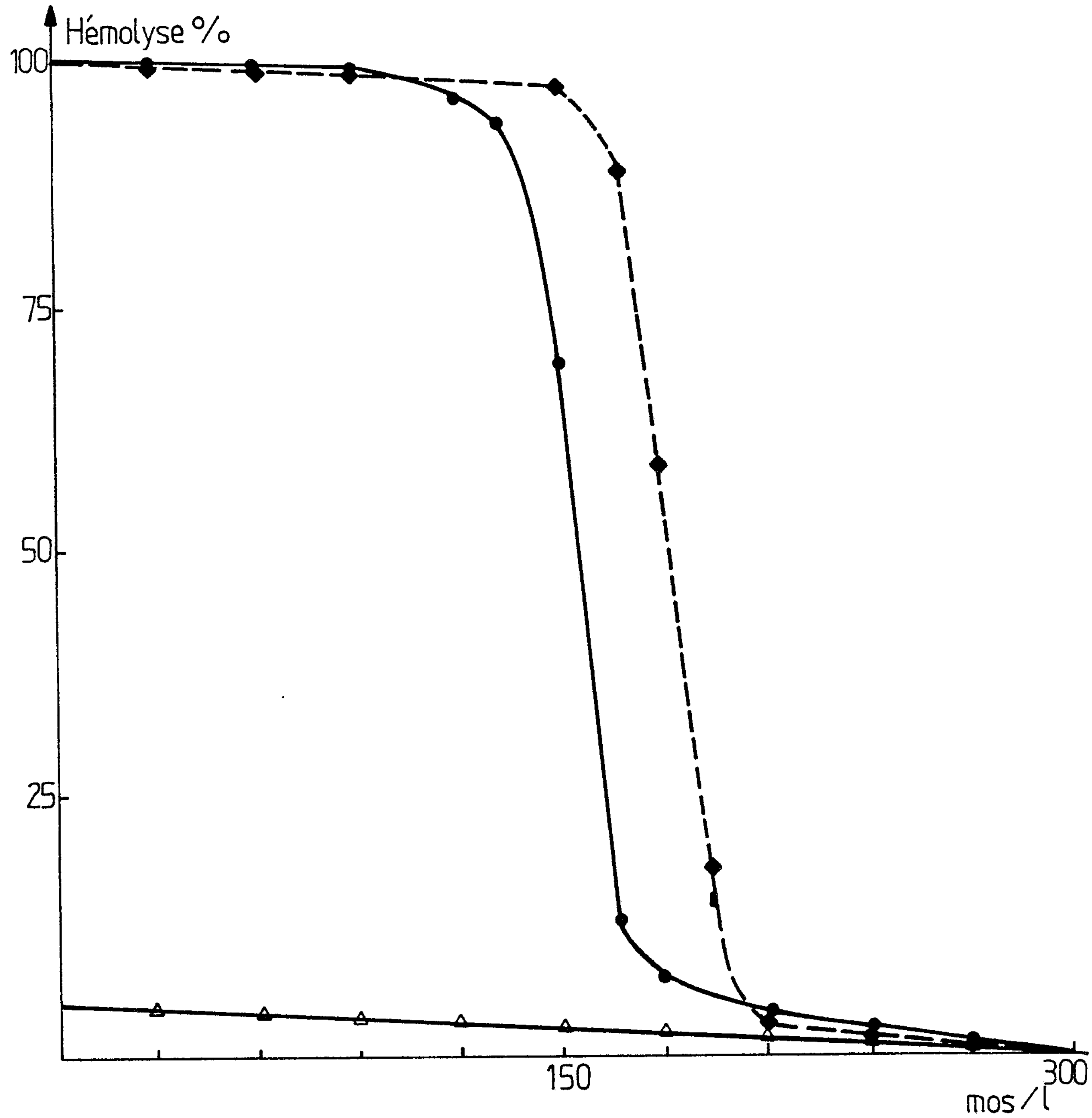

Office européen des brevets

# RAPPORT DE RECHERCHE EUROPEENNE

Numero de la demande

EP 88 40 1608

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | FR-A-2 331 352 (BEHRINGWERKE AG)<br>* Page 2, ligne 9 - page 5, ligne 31 *<br>--- | 1-17 | A 61 K 35/14<br>A 61 K 35/18 |
| Y | WO-A-8 703 484 (BAXTER TRAVENOL LABORATORIES INC.)<br>* Page 2, ligne 27 - page 5, ligne 22 *<br>----- | 1-17 | |
| | | | DOMAINES TECHNIQUES RECHERCHES (Int. Cl.4) |
| | | | A 61 K |

Le présent rapport a été établi pour toutes les revendications

| Lieu de la recherche | Date d'achèvement de la recherche | Examinateur |
|---|---|---|
| LA HAYE | 16-09-1988 | REMPP G.L.E. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)